# EUROPEAN PATENT APPLICATION

(11) **EP 2 762 184 A1**
(43) Date of publication of application: **06.08.2014**
(21) Application number: 13153950.4
(22) Date of filing: 05.02.2013
(51) Int. Cl.: A61M 5/315

(54) **Drive arrangement for drug dispense device**

(71) Applicant: Sanofi-Aventis Deutschland GmbH, 65929 Frankfurt am Main (DE)
(72) Inventor: Ley, Thomas, 65926 Frankfurt am Main (DE)

(57) **Abstract**

A drive arrangement (200) configured to be releasably attachable to a dispense device (100), wherein the drive arrangement (200) is configured to engage with the dispense device (100) for dialing a dose.

## Description

### Field of the invention

The present invention relates to a drive arrangement for a drug dispense device, in particular a pen-type injector, wherein the drive arrangement is configured to engage with the dispense device for dialing a dose and a system comprising a pen-type dispense device and a drive arrangement.

### Background of the invention

For several diseases, medication delivery devices are available to be used by the patient. For example, pen-type devices are used to administer a drug by injection. In a typical situation of use, a patient determines the amount of drug to be injected, sets the corresponding dose size, and injects the set dose. In the area of diabetes e.g., an indication of the required amount of a drug often is provided to the patient by measuring the blood glucose level with a blood glucose meter (BGM). The patient would read the BGM, assume, i.e. calculate or estimate the right dose to be set, set the dose, and finally inject the dose. However, the user may inadvertently set an incorrect dose because he was interrupted after reading the BGM. Or, he may be assuming a dose size that does not correspond to the actual needs. Thus, it would be an improvement to ensure, that the dose that corresponds to a BGM value is actually set in the device. This would help for example, the user to comply with a prescription regime.

In addition, patients suffering from a certain disease may also suffer from side effects of the disease, as for example impaired vision or manual dexterity. Hence, usabilty of device could be improved when providing support means to help the user setting the required dose. Impaired vision, e.g., could lead to setting a false dose because the user could not read the dose dial. Manual dexterity could cause the user not being able to correctly grasp the dose setter or dose dial of an injection device.

US 6,482,185 B1 refers to an injection device with a pin-shaped housing having a manually operated device for setting a dose. The pin-shaped housing is coupled to an external display device that receives information on the respective dose set and displays the dose on a rather large display. Thus, the size of the display device is not limited to the narrow pen shape of the pin-shaped housing. On the other hand, the advantages of the pin-shaped housing, i.e. its being easy to take along and its simple handling, can be maintained. The pin-shaped housing is coupled with the display device only to set the dose. According to another aspect of the invention, the display apparatus sets a dose at the pen. This may be accomplished by providing the display apparatus with a force transmission device to which the pen is connected. If the display apparatus is a blood sugar measuring apparatus, for example, it can calculate the dose from the measured blood sugar rate and further stored parameters, and it can automatically take care that this dose is set at the pen. Thereafter, the pen is removed from the display apparatus.

While overcoming the problems associated with patients suffering from impaired vision, this solution requires at each time of medicament dispense that the user connects the injector to the BGM before setting the dose and disconnect the injector prior to injecting the medicament. The procedure of an injection is thus being extended and complicated. Furthermore, patients suffering from manual dexterity may not be able to perform the additional steps.

It is therefore an objective of the present invention to improve handling of devices used for injection. Furthermore, it is an objective to reduce the manual operation steps in handling devices for injection. Further it is an objective of the present invention to improve safety and compliance with a prescription regime of drug delivery devices in a self-administering environment.

### Summary of the invention

Setting a dose without requiring the user to manually grasp a dose dialer and set the dose, e.g. in rotating the dose dialer could overcome the problems that users experience when having impaired vision that may lead to false dose setting because the display cannot be properly read. Also, setting a dose without requiring the user to manually grasp a dose dialer and set the dose, e.g. in rotating the dose dialer could overcome the problems that user experience when having manual dexterity that may lead to false dose setting because the user is not able to grasp the device.

According to a first aspect, the invention relates to drive arrangement configured to be releasably attachable to a dispense device, wherein the drive arrangement is configured to engage with the dispense device for dialing a dose.

The releasable attachment provides for reuse of the drive arrangement. A user may mount the drive arrangement to a dispense device, e.g. a disposable pen injector. For the life time of the pen-type injector, the drive arrangement will remain mounted and support the user in setting a dose at each injection instance. Once the medicament of the injections device is spent, the user may de-mount the drive arrangement from the injector and mount it to a new one.

The drive arrangement according to the invention comprises a housing having a fastener for mounting the drive arrangement to the dispense device and a drive assembly comprising an actuator configured to engage with at least one of a dose dial and a dose sleeve of the dispense device.

The drive arrangement further comprises a processor and an interface configured for input information, wherein the processor commands the actuator based on the input information for setting a dose.

The drive arrangement may be used with dispense devices of arbitrary shape. In any case, the drive arrangement is arranged to be mounted to the dispense device's housing. The drive arrangement has a drive assembly or drive unit that is configured having engagement structures that correspond to the dose setting means of the dispense device.

For example, the housing of the dispense device may have a basically rectangular shape and a rotatable dose setter on a surface. The drive arrangement of the present invention may be configured to be clipped on the housing. The drive arrangement may have a drive unit comprising a motor that actuates a disc. The disc may be configured to substantially conform to the structures of the dose setter.

In another example, the housing of the dispense device may have a basically pen-shape and a rotatable dose setter. The drive arrangement of the present invention may be configured to be clipped on the housing. Alternatively, the drive arrangement may be configured to embrace the housing. The drive arrangement may have a drive unit comprising a motor that actuates a sleeve. The sleeve may be configured to substantially conform to the structures of the dose setter. Alternatively, the motor may actuate a drive wheel. The drive wheel may be configured to substantially conform to the structures of the dose setter. For example the drive wheel may be biased to provide contact to the dose setter that is sufficient to transmit a force required for moving the dose setter for setting a dose. For example, the drive wheel may be biased to adjust variations of the distance between the dose setter and the drive wheel. For example the drive wheel may made from material, e.g. soft rubber, to provide contact to the dose setter that is sufficient to transmit a force required for moving the dose setter for setting a dose.

According to an embodiment, the actuator comprises a drive wheel and a motor, wherein the drive wheel is driven by the motor. The drive assembly or drive unit further comprises at least one guide wheel, wherein the at least one guide wheel guides the drive wheel to engage with at least one of the dose dial and the dose sleeve such that the dose dial and/or the dose sleeve can be rotated by rotation of the drive wheel.

The drive wheel and the at least on guide wheel are biased in radial direction so that they are pressed against the surface of the dose dial and/or the dose sleeve, thereby adapting the drive assembly to changing diameters.

According to another embodiment, the drive assembly or drive unit is fixed to the housing of the drive arrangement to prevent translation of the drive assembly or drive unit relative to the housing.

In one embodiment, the drive wheel and the at least one guide are configured to allow for translatory slip relative to at least one of the dose dial and the dose sleeve. The dose dial and the dose sleeve perform a combined rotational and translational movement during dose setting. Allowing translatory slip between the drive wheel on one hand and the dose dial and/or the dose sleeve on the other hand could reduce friction of the translatory movement. Thereby the force needed to set the dose is reduced and efficiency of the drive arrangement is improved.

In another embodiment of the drive arrangement, the drive assembly or drive unit is configured to extend proximally together with the dose dial relative to the housing of the drive arrangement.

The drive assembly or drive unit may be fixed to a guiding rod that is mounted to the housing such that the guiding rod can extend telescopically from the housing of the drive arrangement.

In one embodiment, the drive wheel has a drive direction adjusted to the pitch of the thread that guides the movement of the dose dial. Having the drive directions adjusted could reduce friction in directions other than the drive direction. Also, force transmissions from the drive wheel to the dose dial may be optimized because the combined rotational and translational movement of the dose dial and/or dose sleeve is considered.

In a further embodiment of the drive arrangement according to the invention, the interface comprises buttons configured for manual data input.

Alternatively, the interface comprises an electronic data interface coupled to the processor configured for electronic data transfer. This would enable wired or wireless data transfer from another device to the drive arrangement. Hence, the drive arrangement could be controlled remotely.

Further alternatively, the interface comprises a body fluid analyzer or blood glucose monitor coupled to the processor.

According to a second aspect, the invention relates to a portable body fluid analyzer or blood glucose monitor comprising a drive arrangement according to the invention.

According to a third aspect, the invention relates to a system comprising a pen-type dispense device and a drive arrangement according to the invention, wherein the drive arrangement is attached to the dispense device while dispensing a dose of medicament from the dispense device. Having the drive arrangement attached to the dispense device during dispense has the advantage that the user would not have demount it from the dispense device. This may increase user comfort because it reduces the steps to be performed at each instance the user needs to administer medicament. This way, the user may consider that the dispense device and the drive arrangement as a unit. However, having the drive arrangement releasably mounted to the dispense device allows the user remain using a disposable dispense device, that may be known to him and that may provide a known medicament. Thus the user may not have to change medication and/or delivery deivce however could benefit from the advantages of the drive arrangement that supports in setting the dose.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [isoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and p. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the pertinent art that various modifications and variations can be made to the present invention without departing from the spirit and scope of the invention. Further, it is to be noted, that any reference signs used in the appended claims are not to be construed as limiting the scope of the present invention.

Embodiments of the invention will now be described, by way of example only, with reference to the accompanying drawings.

### Brief Description of the Figures

The figures show:
Fig. 1 shows a schematic view of a dispense device of the pen-type;
Fig. 2 show a cross section of a drive arrangement according to the invention attached to the device of figure 1;
Fig. 3 shows the drive arrangement of figure 2 and the device of figure 1 assembled.
Fig. 4 shows a side view section of the drive arrangement of figure 2 and the device of figure 1 assembled before a dose is set
Fig. 5 shows a side view section of the drive arrangement of figure 2 and the device of figure 1 assembled after a dose has been set.
Fig. 6 shows a side view section of another drive arrangement and the device of figure 1 assembled after a dose has been set.

### Detailed Description of Some Embodiments of the Invention

In the following, embodiments of the present invention will be described with reference to an insulin injection device of the pen-type. The present invention is, however, not limited to such application and may equally well be deployed with dispense devices that eject other medicaments, or with other types of medical devices.

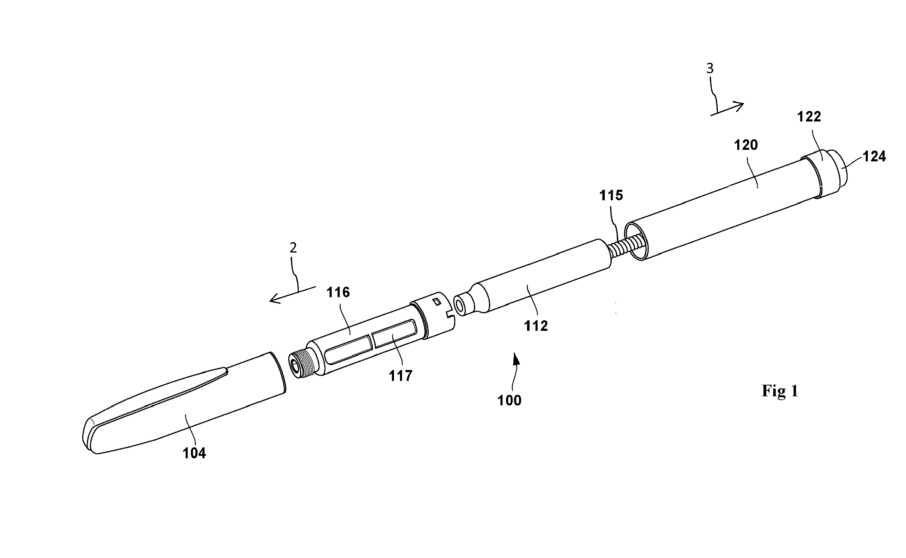

Figure 1 shows one implementation of a dispense device, which is a pen-type injection device 100 having an elongate body 102 comprising a front section 110 and a back section 120, the front section 110 comprising a medicament container, e.g. a cartridge 112 filled with medicament 114, that is fixed to the back section 120 by a cartridge holder 116.

The back section 120 comprises a dose dial 122 for setting the dose and a dispense button 124 to dispense the set dose, both connected to a mechanism (not shown) that is configured to dispense the set dose. A cap 104 is releasably attached to the body covering the front section 110.

A typical mechanism of an injection pen has the dose dial 122 and a dispense button 124 attached to a dose sleeve 126. For setting a dose the dose dial 122 is rotated whereby the dose sleeve 126 also rotates and translates out of the back section 120 of the injection pen 100. The mechanism inside the injection pen 100 determines the movement of the dose dial 122. A helical thread (not shown) of a given pitch guides the movement of the dose dial 122. When the required dose is set, the dose sleeve 126 is pushed back into the back section 120 of the injection pen by pressing the dispense button 124. The mechanism inside the injection pen 100 transmits the translation of the dispense button 124 into a translation of a piston rod 115 that pushes the piston inside the cartridge 112 to displace the medicament 114 out of the cartridge 112.

The cartridge 112 is typically made from glass having a movable piston 113 at one end and a septum 111 at the opposite end.

The cartridge holder 116 may be permanently fixed to the back section 120. Then the injection device is disposable, because once the medicament is spent the device needs to be replaced by a new one.

Alternatively, the cartridge holder 116 is releasably fixed to the back section 120 allowing access to the cartridge (112). Then, the device is reusable, because once the medicament is spent, the cartridge can be replaced and loaded into the device. The cartridge holder 116 is at least partially transparent or has an aperture 117 allowing the user to see the cartridge 112 and/or the medicament 114 inside the cartridge 112.

In a typical situation of use a patient would remove the cap 104 from the device, check the cartridge 112 status, e.g. medicament level, set a required dose in rotating the dose dial 122, mount an injection needle (not shown), and inject the medicament 114 pressing on the dispense button 124 while holding the pen injector 100 against an injection site of his body.

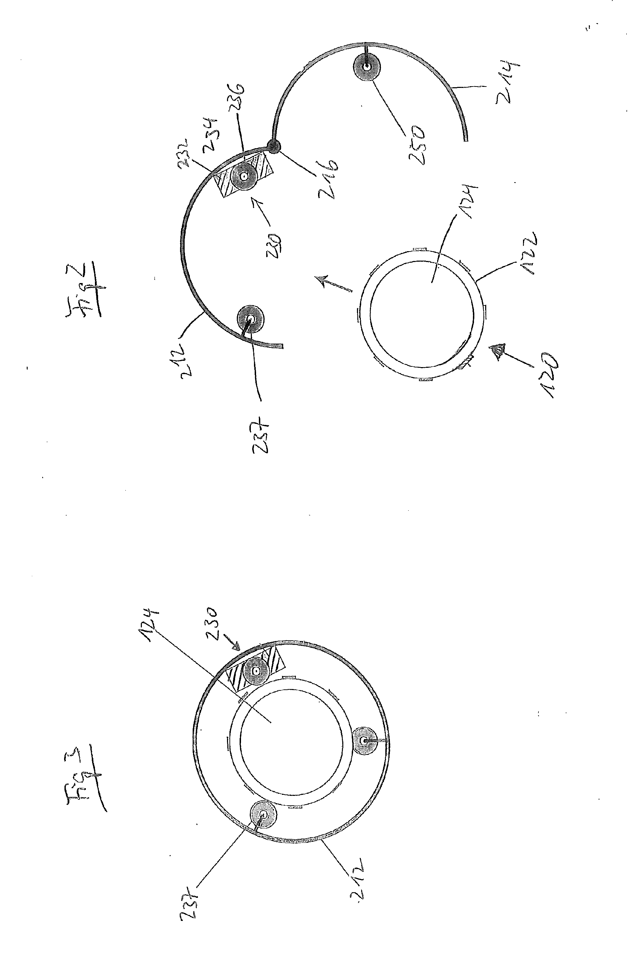

Figure 2 shows a cross section of a drive arrangement according to the invention and a cross section of the device of figure 1.

The drive arrangement 200 has a housing 210 that comprises of two half-shells, 212 and 214, and a connecting joint 216. A first half-shell 212 contains an actuator 230 comprising a motor 232, a gear box 234, and drive wheel 236 as well as a first guide wheel 237. The half-shell 212 further comprises a controller 238 to command the motor 232 and a battery 239 to supply power to the motor 232 and the controller 238. Further, input buttons 240 are arranged on the first shell 212 to set the dose. The other second half-shell 214 comprises a second guide wheel 250. The guide wheels 237 and 250 are biased in radial direction to provide good contact of the guide wheel 237 and 250 with the pen injector 100.

The actuator is also radially inwardly biased.

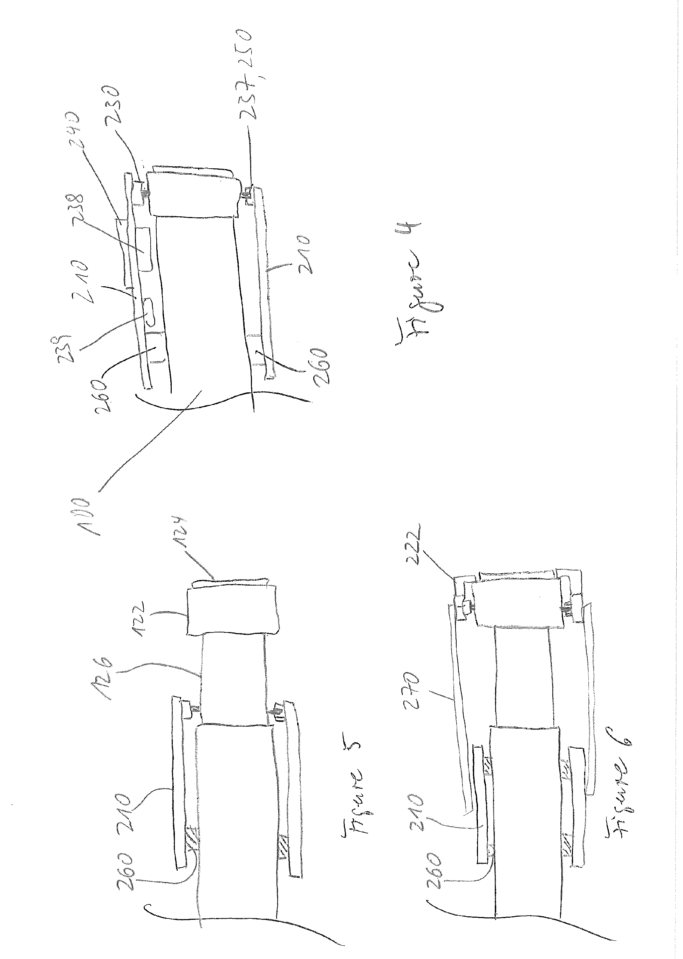

The housing 210 further has a fastener 260 that fixes the drive arrangement to the back section 120 of the pen injector 100 (see Figure 4).

Figure 3 shows a cross section of the drive arrangement 200 of Figure 2 and the pen injector 100 of Figure 2 in an attached state. The two half-shells, 212 and 214, embrace the pen injector 100 at the back section 120. The actuator 230 and the two guide wheels 237, 250 are in contact with the dose dial 122 of the pen injector 100.

Figure 4 shows a section view of the drive arrangement 200 mounted on the back section 120 of the pen injector 100 before dose setting. The housing 210 of the drive arrangement 200 is positioned on the pen injector 100 such that the drive wheel 236 and guide wheels 237, 250 engage with the dose dial 122 and the fasteners 260 engage with the back section 120 of the pen injector 100.

During dose setting, when the motor 232 rotates the drive wheel 236, the dose dial 122 is rotated and a dose is set. While the motor 232 rotates the dose dial 122, it is rotated out of the pen injector 100 in proximal direction (arrow 3, Figure 1) according to the mechanism of the pen injector 100. Also the dose sleeve 126 also rotates out of the housing of the pen injector 100.Typically, the dose sleeve 126 has a smaller diameter than the dose dial 122. The guide wheels 237, 250 can be moved in radial direction and are radially inwardly biased so that they are pressed against the surface of the dose dial 122 and/or the dose sleeve 126, thereby adapting the drive assembly 220 to changing diameters.

Figure 5 schematically shows a section view of Figure 4. The dose dial 122 is rotated out which corresponds to a position when the dose is set. The drive arrangement is still in place mounted on the back section 120 of the pen injector 100 as the fasteners 260 provide a connection for securely fixing the drive arrangement on the pen injector. The drive wheel 236 and guide wheels 237, 250 are in engagement with the dose sleeve 126.

The drive wheel 236 of this drive arrangement 200 is made from soft rubber to provide good transmission from the rotating drive wheel 236 to the dose dial 122 and /or dose sleeve 126. The guide wheels 237, 250 are made from hard rubber to provide good guidance however provide low friction. In fact, slippery material could be used because this reduces friction as the dose dial 122 and/or dose sleeve 126 rotate and thereby translate too. Thus, the drive wheel 236 and/or the guide wheels 237, 250 allow for translatory slip relative the dose dial 122 and/or the dose sleeve 126.

In a further embodiment (not shown) the drive wheel is adjusted to the pitch of the thread that guides the movement of the dose dial 122. This embodiment preferably features a soft rubber drive wheel 236 to ensure transmission of the rotary movement.

In an alternative embodiment shown in Figure 6, the drive arrangement 200 has a drive assembly or drive unit 220 comprising the actuator 230 and the guide wheels 237, 250.The drive assembly 220 is fixed to a guiding rod 270 that is mounted to the housing 210 of the drive arrangement 200 such that the guiding rod 270 and with it the drive assembly 220 can extend telescopically from the housing 210 of the drive arrangement 200.

The drive arrangement 200 has extensions 222 that abut against the distal step of the dose dial 122. During dose setting, as described above, the dose wheel it is rotated out of the pen injector 100 in proximal direction (arrow 3, Figure 1) according to the mechanism of the pen injector 100. Also the dose sleeve 126 rotates out of the housing of the pen injector 100. However, as the extensions of the drive assembly 220 engage with the proximal step at the dose dial 122 the translatory movement of the dose dial 122 is transmitted to the drive assembly. Hence the drive assembly moves along with the dose dial 122 during dose setting while the the guiding rod 270 and with it the drive assembly 220 extend telescopically from the housing 210 of the drive arrangement 200.

The guiding rod 270 provides fixation of the drive arrangement 200 relative to the housing of the pen injector 100 thereby enabling that the rotation of the drive wheel 236 is transmitted into rotation of the dose dial 122. The guiding rod 270 therefore is designed to withstand against torque. As shown in Figure 6, the embodiment feature two guiding rods 270. Alternatively the guiding rods 270 could be replaced by a sleeve or a set of telescoping sleeves. The drive wheel 236 of this embodiment is preferably made from soft rubber as outlined in the previous embodiment. Preferably the drive wheel is aligned in an angle perpendicular to the length axis of the pen injector 100. As there is no relative translatory movement between the guide wheels 237, 250 and the dose dial 122 and/or the dose sleeve 126 the guide wheels 237, 250 can be made from soft rubber too.

In an alternative embodiment, the drive arrangement 200 has an electrical interface (not shown) instead of the input buttons 240. The electrical interface can receive information from a remote device for setting a dose. For example, the remote device could be a

In a further alternative embodiment, the drive arrangement 200 comprises a BGM integrated with the drive arrangement wherein the BGM is connected to the controller 238 of the drive arrangement 200.

### List of reference numbers

- 100: dispense device, pen-type injection device
- 102: elongate body
- 104: cap
- 110: front section
- 111: septum
- 112: medicament container, e.g. a cartridge
- 113: piston
- 114: medicament
- 115: piston rod
- 116: cartridge holder
- 117: aperture
- 120: back section
- 122: dose dial
- 124: dispense button
- 126: dose sleeve

- 200: drive arrangement
- 210: housing
- 212: first half-shell
- 214: second half-shell
- 216: connecting joint
- 220: drive assembly or drive unit
- 222: extensions
- 230: actuator
- 232: motor
- 234: gear box
- 236: drive wheel
- 237: first guide wheel
- 238: controller, processor
- 239: battery
- 240: input buttons, interface
- 250: second guide wheel
- 260: fastener
- 270: guiding rod

## Claims

1. A drive arrangement (200) configured to be releasably attachable to a dispense device (100), wherein the drive arrangement (200) is configured to engage with the dispense device (100) for dialing a dose.

2. A drive arrangement (200) according to claim 1, wherein the drive arrangement (200) comprises a housing (210) having a fastener (260) for mounting the drive arrangement (200) to the dispense device (100) and a drive assembly (220) comprising an actuator (230) configured to engage with at least one of a dose dial (122) and a dose sleeve (126) of the dispense device (100).

3. A drive arrangement (200) according to claim 2, wherein the drive arrangement (200) further comprises, a processor (238), and an interface (240) configured for input information, wherein the processor (238) commands the actuator (230) based on the input information for setting a dose.

4. A drive arrangement (200) according to claim 2 or 3, wherein the actuator (230) comprises a drive wheel (236) and a motor (232), wherein the drive wheel (236) is driven by the motor (232), and wherein the drive assembly (220) further comprises at least one guide wheel (237, 250), wherein the at least one guide wheel (237, 250) guides the drive wheel (236) to engage with at least one of the dose dial (122) and the dose sleeve (126) such that the dose dial (122) and/or the dose sleeve (126) can be rotated by rotation of the drive wheel (236).

5. A drive arrangement (200) according to claim 4, wherein
the drive wheel (236) and the at least on guide wheel (237, 250) are biased in radial direction so that they are pressed against the surface of the dose dial (122) and/or the dose sleeve (126), thereby adapting the drive assembly (220) to changing diameters.

6. A drive arrangement (200) according to any of claims 2 to 5, wherein
the drive assembly (220) is fixed to the housing (210) to prevent translation of the drive assembly (220) relative to the housing (210).

7. A drive arrangement (200) according to claim 6, wherein
the drive wheel (236) and the at least one guide (237, 250) allow for translatory slip relative to at least one of the dose dial (122) and the dose sleeve (126).

8. A drive arrangement (200) according to any of claim 2 to 5, wherein
the drive assembly (220) is configured to extend proximally together with the dose dial (122) relative to the housing (210) of the drive arrangement (200).

9. A drive arrangement (200) according to claim 8, wherein the drive assembly (220) is fixed to a guiding rod (270) that is mounted to the housing (210) such that the guiding rod (270) can extend telescopically from the housing (210) of the drive arrangement (200).

10. A drive arrangement (200) according to claim 8 or 9, wherein the drive wheel (236) has a drive direction adjusted to the pitch of the thread that guides the movement of the dose dial (122).

11. A drive arrangement (200) according to any preceding claim, wherein the interface (240) comprises buttons configured for manual data input.

12. A drive arrangement (200) according to any of claims 1 through 9, wherein the interface (240) comprises an electronic data interface coupled to the processor (238) configured for electronic data transfer.

13. A drive arrangement (200) according to any of claims 1 through 10, wherein the interface (240) comprises a body fluid analyzer or blood glucose monitor coupled to the processor (238).

14. A portable body fluid analyzer comprising a drive arrangement (200) according to any of claims 1 through 12.

15. A system comprising a pen-type dispense device (100) and a drive arrangement (200) according to any preceding claim, wherein the drive arrangement (200) is attached to the delivery device (100) while dispensing a dose of medicament from the dispense device (100).
